# EUROPEAN PATENT APPLICATION

(11) **EP 4 471 142 A1**
(43) Date of publication of application: **04.12.2024**
(21) Application number: 22923647.6
(22) Date of filing: 29.12.2022
(51) Int. Cl.: C12N 15/113, C12N 1/21, C12N 15/77, C12P 13/08, C12R 1/15

(54) **RECOMBINANT MICROORGANISM FOR PRODUCING THREONINE AND USE THEREOF**

(30) Priority: 28.01.2022 CN 202210107366
(71) Applicant: Langfang Meihua Biotechnology Development Co., Ltd, Langfang City, Hebei 065001 (CN)
(72) Inventor: KANG, Pei, Langfang City, Hebei 065001 (CN); WU, Tao, Langfang City, Hebei 065001 (CN); GONG, Weibo, Langfang City, Hebei 065001 (CN); HE, Jun, Langfang City, Hebei 065001 (CN); LI, Yan, Langfang City, Hebei 065001 (CN)
(74) Representative: CH Kilger Anwaltspartnerschaft mbB
(86) International application number: PCT/CN2022/143106
(87) International publication number: WO 2023/142862

(57) **Abstract**

Provided are a recombinant microorganism for producing threonine and the use thereof in the fermentation production of threonine or a derivative thereof. A 20-30 bp segment upstream of a start codon of a gene encoding phosphoenolpyruvate carboxylase in the recombinant microorganism is replaced with a strong promoter. By means of the specific optimization of the promoter of the gene encoding phosphoenolpyruvate carboxylase and the mutation of the encoding region of the gene, the ability of the strain to synthesize threonine is significantly improved.

## Description

### Technical Field

The present invention relates to the technical field of microbial engineering, specifically to a recombinant microorganism for producing threonine and use thereof.

### Background Art

L-Threonine (chemically known as β-hydroxy-α-aminobutyric acid) has a molecular formula of C₄H₉NO₃, a relative molecular mass of 119.12, and is an essential amino acid, mainly used in medicine, chemical reagents, food fortifiers, feed additives, etc.

In Corynebacterium glutamicum, the production of threonine from oxaloacetate requires a five-step catalytic reaction, the catalytic enzymes of which are aspartate kinase (encoded by lysC), aspartate semialdehyde dehydrogenase (encoded by asd), homoserine dehydrogenase (encoded by horn), homoserine kinase (encoded by thrB) and threonine synthase (encoded by thrC), respectively. Current reports on using Corynebacterium glutamicum to produce threonine mainly focus on the modification of the anabolic pathway of threonine, including: obtaining feedback-resistant homoserine dehydrogenase and aspartate kinase (Reinscheid D J, Eikmanns B J, Sahm H. Analysis of a Corynebacterium glutamicum horn gene coding for a feedback-resistant homoserine dehydrogenase.[J]. Journal of Bacteriology, 1991, 173(10):3228-3230; Eikmanns B J, Eggeling L, Sahm H. Molecular aspects of lysine, threonine, and isoleucine biosynthesis in Corynebacterium glutamicum.[J]. Antonie Van Leeuwenhoek, 1993, 64(2):145-163.); and attenuating the coding gene glyA in the threonine utilization pathway and overexpressing the threonine export protein ThrE (Simic P, Willuhn J, Sahm H, et al. Identification of glyA (Encoding Serine Hydroxymethyltransferase) and Its Use Together with the Exporter ThrE To Increase l-Threonine Accumulation by Corynebacterium glutamicum[J]. Applied and Environmental Microbiology, 2002, 68(7):3321-3327), etc. However, there are still few reports on the supply of precursors for threonine production.

### Summary of the Invention

The objective of the present invention is to provide a recombinant microorganism for producing threonine by modifying a promoter region of a gene encoding phosphoenolpyruvate carboxylase to improve the threonine-producing ability of a strain, and use of the recombinant microorganism.

Specifically, the present invention provides the following technical solutions:
First, the present invention provides use of improved expression of the gene encoding phosphoenolpyruvate carboxylase (ppc) in increasing the threonine production of Corynebacterium bacteria or constructing threonine producing Corynebacterium bacteria, wherein the improved expression is achieved by replacing 20-30 bp upstream of the start codon of the gene encoding phosphoenolpyruvate carboxylase with a strong promoter.

The Corynebacterium bacteria described above are preferably Corynebacterium glutamicum.

The present invention finds that in Corynebacterium glutamicum, increasing the enzymatic activity of phosphoenolpyruvate carboxylase is beneficial to increasing the supply of oxaloacetate, a precursor of threonine synthesis, and further promoting the synthesis and accumulation of threonine. With regard to methodes to increase the enzyme activity of phosphoenolpyruvate carboxylase, the present invention has conducted a large number of attempts and screenings, and unexpectedly found that replacing the specific region in the noncoding region upstream of the gene encoding phosphoenolpyruvate carboxylase with a strong promoter can significantly increase the expression quantity of phosphoenolpyruvate carboxylase. Compared to increasing the copy number of the gene, this modification method has higher stability. Moreover, compared to replacing the promoter of ppc with a stronger promoter, this modification is significantly better in improving the enzyme activity of phosphoenolpyruvate carboxylase.

Preferably, the enhanced expression is achieved by replacing 27 bp upstream of the start codon of the gene encoding phosphoenolpyruvate carboxylase with a strong promoter.

Further preferably, the strong promoter is Ptuf. The nucleotide sequence of the strong promoter Ptuf is as shown in SEQ ID NO. 4.

In order to enhance gene expression, a strong promoter is often used to replace the original promoter of the gene or directly inserted upstream of the start codon of the gene. When replacing the promoter, all the spacer sequence between the gene of interest and the upstream gene is usually replaced with a strong promoter, or the original promoter region of the gene of interest is replaced with a strong promoter. However, the present invention found that replacing the 27 bp DNA segment upstream of the start codon with a strong promoter (especially the strong promoter Ptuf) is significantly better in increasing the expression quantity of phosphoenolpyruvate carboxylase than replacing the promoter region of the gene encoding phosphoenolpyruvate carboxylase.

The 27 bp segment upstream of the start codon of the present invention means that: the first base upstream of the start codon is taken as the base at position 1 and extended upstream to the base at postition 27 to obtain a DNA segment with a length of 27 bp. Taking the wild-type strain of Corynebacterium glutamicum ATCC13032 as an example, the sequence of the 27 bp segment upstream of the start codon of the gene encoding phosphoenolpyruvate carboxylase is as shown in SEQ ID NO.3.

The amino acid sequence of the phosphoenolpyruvate carboxylase of the present invention is shown in SEQ ID NO.1 or 2, or an amino acid sequence having at least 90% similarity to and having equivalent functions as the sequence shown in SEQ ID NO.1 or 2.

Preferably, the amino acid sequence of phosphoenolpyruvate carboxylase is as shown in SEQ ID NO. 1 or 2.

The sequence shown in SEQ ID NO.1 is the amino acid sequence of wild-type phosphoenolpyruvate carboxylase of Corynebacterium glutamicum. The sequence shown in SEQ ID NO.2 is a mutant protein (D299N) obtained by mutating the D at position 299 to N based on the wild-type phosphoenolpyruvate carboxylase of Corynebacterium glutamicum. On the basis of the above modification of the promoter region, combined with the mutation D299N of phosphoenolpyruvate carboxylase, the threonine production of the strain can be significantly improved. The amino acid sequence of phosphoenolpyruvate carboxylase is as shown in SEQ ID NO. 2.

Further, the present invention provides a recombinant microorganism, in which the 20-30 bp segment upstream of an start codon of the gene encoding phosphoenolpyruvate carboxylase is replaced with a strong promoter.

Preferably, a 27 bp segment upstream of the start codon of the gene encoding phosphoenolpyruvate carboxylase in the recombinant microorganism is replaced with a strong promoter.

Further preferably, the strong promoter is Ptuf.

The amino acid sequence of the phosphoenolpyruvate carboxylase described above is shown in SEQ ID NO.1 or 2, or an amino acid sequence having at least 90% similarity to and having equivalent functions as the sequence shown in SEQ ID NO.1 or 2.

Preferably, the amino acid sequence of phosphoenolpyruvate carboxylase is as shown in SEQ ID NO. 1 or 2. On the basis of the above modification of the promoter region, combined with the mutation D299N of phosphoenolpyruvate carboxylase, the threonine production of the strain can be significantly improved. Therefore, it is preferable that the amino acid sequence of the phosphoenolpyruvate carboxylase of the recombinant microorganism is as shown in SEQ ID NO.2.

The recombinant microorganism described above is preferably constructed by the following method: in the original strain, the 27 bp segment upstream of the start codon of the gene encoding phosphoenolpyruvate carboxylase of the original strain is replaced with a strong promoter Ptuf. Preferably, the method further comprises the step of mutating the gene encoding phosphoenolpyruvate carboxylase so that the phosphoenolpyruvate carboxylase obtains the D299N mutation.

Among them, the original strain is preferably a strain that can accumulate threonine.

Preferably, the enzyme activity of any one or more of the following enzymes (1) to (7) is enhanced and/or the feedback inhibition thereof is deregulated in the recombinant microorganism:
(1) aspartate kinase;
(2) aspartate semialdehyde dehydrogenase;
(3) homoserine dehydrogenase;
(4) threonine synthase;
(5) homoserine kinase;
(6) aspartate aminotransferase;
and (7) threonine export protein;

Preferably, the threonine export protein is a threonine export protein derived from Escherichia coli.

The above-mentioned aspartate kinase, homoserine dehydrogenase, aspartate semialdehyde dehydrogenase, aspartate aminotransferase, homoserine kinase, and threonine synthase have reference sequence numbers of WP_003855724.1, WP_003854900.1, WP_011013506.1, WP_011013497.1, WP_011014183.1, and WP_011014964.1 on NCBI, respectively, or amino acid sequences with at least 90% similarity to and equivalent functions as the above-mentioned reference sequences.

The threonine export protein derived from Escherichia coli has a reference sequence number of YP_026264.1 on NCBI, or an amino acid sequence with at least 90% similarity to and equivalent function as the above reference sequence.

Preferably, the microorganism is any of the following (1) to (6):
(1) a microorganism in which the 20-30 bp segment upstream of the start codon of the gene encoding phosphoenolpyruvate carboxylase is replaced with a strong promoter, and the activity of aspartate aminotransferase is enhanced and/or the feedback inhibition thereof is deregulated;
(2) a microorganism in which the 20-30 bp segment upstream of the start codon of the gene encoding phosphoenolpyruvate carboxylase is replaced with a strong promoter, and the activity of aspartate aminotransferase, aspartate kinase and/or aspartate semialdehyde dehydrogenase is enhanced and/or the feedback inhibition thereof is deregulated;
(3) a microorganism in which the 20-30 bp segment upstream of the start codon of the gene encoding phosphoenolpyruvate carboxylase is replaced with a strong promoter, and the activity of aspartate aminotransferase, aspartate kinase, aspartate semialdehyde dehydrogenase and/or homoserine dehydrogenase is enhanced and/or the feedback inhibition thereof is deregulated;
(4) a microorganism in which the 20-30 bp segment upstream of the start codon of the gene encoding phosphoenolpyruvate carboxylase is replaced with a strong promoter, and the activity of aspartate aminotransferase, aspartate kinase, aspartate semialdehyde dehydrogenase, homoserine dehydrogenase and/or homoserine kinase is enhanced and/or the feedback inhibition thereof is deregulated;
(5) a microorganism in which the 20-30 bp segment upstream of the start codon of the gene encoding phosphoenolpyruvate carboxylase is replaced with a strong promoter, and the activity of aspartate aminotransferase, aspartate kinase, aspartate semialdehyde dehydrogenase, homoserine dehydrogenase, homoserine kinase and/or threonine synthase is enhanced and/or the feedback inhibition thereof is deregulated;
(6) a microorganism in which the 20-30 bp segment upstream of the start codon of the gene encoding phosphoenolpyruvate carboxylase is replaced with a strong promoter, and the activity of aspartate aminotransferase, aspartate kinase, aspartate semialdehyde dehydrogenase, homoserine dehydrogenase, homoserine kinase, threonine synthase and/or threonine export protein is enhanced and/or the feedback inhibition thereof is deregulated.

The enhancement of the activity of the above enzymes is achieved by any one selected from the following 1) to 6), or an optional combination thereof:
1) enhancement by introducing a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme;
and 6) enhancement by altering the nucleotide sequence encoding the enzyme.

As a preferred scheme of the present invention, the recombinant microorganism is any of the following (1)-(6):
(1) a microorganism in which a mutation site D299N is introduced into the phosphoenolpyruvate carboxylase and a 27 bp segment upstream of the start codon of the coding gene is replaced with a Ptuf promoter, and at the same time, the expression of aspartate aminotransferase is enhanced;
(2) a microorganism in which a mutation site D299N is introduced into phosphoenolpyruvate carboxylase and a 27 bp segment upstream of the start codon of the coding gene is replaced with a Ptuf promoter, and at the same time, the expression of aspartate aminotransferase, aspartate kinase, and aspartate semialdehyde dehydrogenase is enhanced, and the feedback inhibition thereof is deregulated;
(3) a microorganism in which a mutation site D299N is introduced into phosphoenolpyruvate carboxylase and a 27 bp segment upstream of the start codon of the coding gene is replaced with a Ptuf promoter, and at the same time, the expression of aspartate aminotransferase, aspartate kinase, aspartate semialdehyde dehydrogenase and homoserine dehydrogenase is enhanced, and the feedback inhibition of aspartate kinase and homoserine dehydrogenase is deregulated;
(4) a microorganism in which a mutation site D299N is introduced into phosphoenolpyruvate carboxylase and a 27 bp segment upstream of the start codon of the coding gene is replaced with a Ptuf promoter, and at the same time, the expression of aspartate aminotransferase, aspartate kinase, aspartate semialdehyde dehydrogenase, homoserine dehydrogenase, and homoserine kinase is enhanced, and the feedback inhibition of aspartate kinase and homoserine dehydrogenase is deregulated;
(5) a microorganism in which a mutation site D299N is introduced into phosphoenolpyruvate carboxylase and a 27 bp segment upstream of the start codon of the coding gene is replaced with a Ptuf promoter, and at the same time, the expression of aspartate aminotransferase, aspartate kinase, aspartate semialdehyde dehydrogenase, homoserine dehydrogenase, homoserine kinase and threonine synthase is enhanced, and the feedback inhibition of aspartate kinase and homoserine dehydrogenase is deregulated;
(6) a microorganism in which a mutation site D299N is introduced into phosphoenolpyruvate carboxylase and a 27 bp upstream of the start codon ATG of the coding gene is replaced with a Ptuf promoter, and the expression of aspartate aminotransferase, aspartate kinase, aspartate semialdehyde dehydrogenase, homoserine dehydrogenase, homoserine kinase and threonine synthase is enhanced, the threonine export protein derived from Escherichia coli was expressed, and the feedback inhibition of aspartate kinase and homoserine dehydrogenase is deregulated.

Preferably, the above-mentioned enhanced enzyme activity or enhanced expression is achieved by replacing the original promoter of the gene with a strong promoter, or by mutating the start codon of the gene so that the amino acid at position 1 of the encoded protein is mutated from valine to methionine.

The strong promoter is preferably the promoter Psod, PcspB or Ptuf. Among them, the nucleotide sequences of promoters Psod and PcspB are as shown in SEQ ID NOs. 5 and 6, respectively.

Preferably, the enhanced expression of the gene encoding aspartate kinase and the gene encoding threonine synthase is achieved by replacing their original promoter with promoter Psod and their start codon GTG was replaced by ATG.

The enhanced expression of the gene encoding aspartate semialdehyde dehydrogenase and the gene encoding aspartate aminotransferase is achieved by replacing their original promoters with the promoter Psod.

The enhanced expression of the gene encoding homoserine dehydrogenase and the gene encoding homoserine kinase is achieved by replacing their original promoters with the promoter PcspB.

The expression of the threonine export protein of Escherichia coli can be achieved by integrating the expression cassette of the gene encoding the threonine export protein of Escherichia coli into the chromosome of the microorganism. The preferred integration site is downstream of the cg2009 gene, and the promoter of the expression cassette is Psod.

The feedback-resistant aspartate kinase described above is preferably achieved by the T311I mutation. The feedback-resistant homoserine dehydrogenase is preferably achieved by the G378E mutation.

The microorganism used to construct the recombinant microorganism described above is preferably a Corynebacterium bacterium, more preferably Corynebacterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

The present invention further provides a method for constructing the recombinant microorganism described above, comprising: the 27 bp segment upstream of the start codon of the gene encoding phosphoenolpyruvate carboxylase of the original strain is replaced with a strong promoter Ptuf.

Preferably, the method further comprises mutating the gene encoding phosphoenolpyruvate carboxylase so that the phosphoenolpyruvate carboxylase got the D299N mutation.

Further preferably, the method further comprises: enhancing the enzyme activity and/or deregulating the feedback inhibition of any one or more of the following enzymes (1) to (7):
(1) aspartate kinase;
(2) aspartate semialdehyde dehydrogenase;
(3) homoserine dehydrogenase;
(4) threonine synthase;
(5) homoserine kinase;
(6) aspartate aminotransferase;
and (7) threonine export protein;
wherein, the enhancement of the enzyme activity is achieved by any one selected from the following 1) to 6), or an optional combination thereof:
   1) enhancement by introducing a plasmid having the gene encoding the enzyme;
   2) enhancement by increasing the copy number of the gene encoding the enzyme on the chromosome;
   3) enhancement by altering the promoter sequence of the gene encoding the enzyme on the chromosome;
   4) enhancement by operably linking a strong promoter to the gene encoding the enzyme;
   5) enhancement by altering tthe amino acid sequence of the enzyme;
   and 6) enhancement by altering the nucleotide sequence encoding the enzyme.

The above-mentioned modification methods, including gene enhancement, are all modification methods known to those skilled in the art, see MAN, Zaiwei. Systemic pathway engineering modification of Corynebacterium crenatum to produce L-arginine with high yield [D]. Jiangnan University, 2016; CUI, Yi. Metabolic engineering modification of Corynebacterium glutamicum to produce L-leucine [D]. Tianjin University of Science and Technology.; Xu Guodong. Construction of L-isoleucine producing strain and optimization of fermentation conditions. Tianjin University of Science and Technology, 2015.

The present invention provides one of the following uses of the recombinant microorganism described above:
(1) use in fermentation of threonine or a derivative thereof;
(2) use in constructing a threonine or a derivative thereof producing strain as an original strain;
and (3) use in increasing the production and/or yield of threonine or a derivative thereof.

The present invention further provides a method for producing threonine or a derivative thereof by fermentation, the method comprises the steps of cultivating the recombinant microorganism described above and obtaining threonine or the derivative thereof by isolating threonine or the derivative thereof from the culture.

Specifically, the above method comprises that: the recombinant microorganism is inoculated in a seed medium to obtain a seed liquid, the seed liquid is inoculated in a fermentation medium to obtain a fermentation liquid, and the fermentation liquid is separated and extracted to obtain threonine or a derivative thereof.

Preferably, the fermentation medium comprises the following components: corn steep liquor 45-55 mL/L, glucose 25-35 g/L, ammonium sulfate 3-5 g/L, MOPS 25-35 g/L, potassium dihydrogen phosphate 8-12 g/L, urea 15-25 g/L, biotin 8-12 mg/L, magnesium sulfate 5-7 g/L, ferrous sulfate 0.5-1.5 g/L, VB1•HCl 35-45 mg/L, calcium pantothenate 45-55 mg/L, nicotinamide 35-45 mg/L, manganese sulfate 0.5-1.5 g/L, zinc sulfate 15-25 mg/L, copper sulfate 15-25 mg/L, pH 7.0-7.2.

The beneficial effects of the present invention are as follows: the present invention significantly improved the ability of the strain to synthesize threonine by means of the specific optimization of the promoter of the gene encoding phosphoenolpyruvate carboxylase and the mutation of the encoding region of the gene, and the threonine production is increased by 25-40% compared to the strain without the above modification. The above modification method can be applied to the construction of threonine-producing strains and fermentative production of threonine, and has good application value.

### Detailed Description of Embodiments

The following examples are intended to illustrate the present invention but are not intended to limit the scope of the present invention.

The present invention focuses on investigating the impact of modification of the promoter region of phosphoenolpyruvate carboxylase on threonine production. It has been verified that replacing 20-30 bp, preferably 27 bp, upstream of the start codon of the gene encoding phosphoenolpyruvate carboxylase with a strong promoter can significantly increase the production of threonine.

The present invention further strengthens the threonine synthesis and transport pathways of the strain, mainly including expression enhancement or deregulating of aspartate kinase, homoserine dehydrogenase, aspartate semialdehyde dehydrogenase, aspartate aminotransferase, homoserine kinase, threonine synthase, and threonine export protein. The results of the shake flasks showed that the threonine-producing ability of all threonine-producing strains was improved after the 20-30 bp upstream of the start codon of the gene encoding phosphoenolpyruvate carboxylase was replaced with a strong promoter. At the same time, compared to the strain in which only the 20-30 bp upstream of the start codon of the gene encoding phosphoenolpyruvate carboxylase was replaced with a strong promoter, the strain in which the 20-30 bp upstream of the start codon of the gene encoding phosphoenolpyruvate carboxylase was replaced with a strong promoter and the expression of enzymes involved in the threonine synthesis and transport pathways was enhanced had more advantages in the production of threonine.

Expression enhancement during the modification process includes methods such as promoter replacement, change of ribosome binding sites, increase in copy number, and plasmid overexpression, and the above means are all well known to researchers in the art. The above means cannot be exhaustive through examples, and the specific embodiments only use enhancement by promoter as a representative for illustration.

The present invention adopts the following technical solutions:

A first technical solution of the present invention provides a method for producing threonine by a strain in which the 20-30 bp segment upstream of the start codon of the gene encoding phosphoenolpyruvate carboxylase is replaced with a strong promoter, and the expression of aspartate aminotransferase is enhanced and/or deregulated.

A second technical solution of the present invention provides a method for producing threonine by a strain in which the 20-30 bp segment upstream of the start codon of the gene encoding phosphoenolpyruvate carboxylase is replaced with a strong promoter, and the expression of at least one of aspartate aminotransferase, aspartate kinase, and aspartate semialdehyde dehydrogenase is enhanced and/or deregulated.

A third technical solution of the present invention provides a method for producing threonine by a strain in which the 20-30 bp segment upstream of the start codon of the gene encoding phosphoenolpyruvate carboxylase is replaced with a strong promoter, and the expression of at least one of aspartate aminotransferase, aspartate kinase, aspartate semialdehyde dehydrogenase, and homoserine dehydrogenase is enhanced and/or deregulated.

A fourth technical solution of the present invention provides a method for producing threonine by a strain in which the 20-30 bp segment upstream of the start codon of thegene encoding phosphoenolpyruvate carboxylase is replaced with a strong promoter, and the expression of at least one of aspartate aminotransferase, aspartate kinase, aspartate semialdehyde dehydrogenase, homoserine dehydrogenase, and homoserine kinase is enhanced and/or deregulated.

A fifth technical solution of the present invention provides a method for producing threonine by a strain in which the 20-30 bp segment upstream of the start codon of the gene encoding phosphoenolpyruvate carboxylase is replaced with a strong promoter, and the expression of at least one of aspartate aminotransferase, aspartate kinase, aspartate semialdehyde dehydrogenase, homoserine dehydrogenase, homoserine kinase, and threonine synthase is enhanced and/or deregulated.

A sixth technical solution of the present invention provides a method for producing threonine by a strain in which the 20-30 bp segment upstream of the start codon of the gene encoding phosphoenolpyruvate carboxylase is replaced with a strong promoter, and the expression of at least one of aspartate aminotransferase, aspartate kinase, aspartate semialdehyde dehydrogenase, homoserine dehydrogenase, homoserine kinase, threonine synthase, and threonine export protein is enhanced and/or deregulated.

The above-mentioned strain is a Corynebacterium bacterium, preferably Corynebacterium glutamicum, and most preferably Corynebacterium glutamicum ATCC 13032.

The detailed information of the enzymes and genes involved in the present invention are as follows:
aspartate kinase, coding gene name lysC, NCBI number: cg0306, Cgl0251, NCgl0247;
aspartate semialdehyde dehydrogenase, coding gene name asd, NCBI number: Cgl0252, Cg0307, NCgl0248;
homoserine dehydrogenase, coding gene name horn, NCBI number: Cg1337, Cgl1183, NCgl1136;
threonine synthase, coding gene name thrC, NCBI number: cg2437, Cgl2220, NCgl2139;
homoserine kinase, coding gene thrB, NCBI number: Cgl1184, Cg1338, NCgl1137;
phosphoenolpyruvate carboxylase, coding gene ppc, NCBI number: cg1787, Cgl1585, NCgl1523;
aspartate aminotransferase, coding gene name aspB, NCBI number: cg0294, cg0294 and cg0294;
Escherichia coli-derived threonine export protein, encoding gene name rhtC, NCBI number: 948317.

### Example 1 Construction of plasmids for genome modification of strains

### 1. Construction of the plasmid pK18mobsacB-P_{sod}-lysC^{gla-T311I}-asd for enhancing expression of aspartate kinase-aspartate semialdehyde dehydrogenase operon

The upstream homologous arm up was obtained by PCR amplification with P21/P22 primer pair using ATCC 13032 genome as template, the promoter segment Psod was obtained by PCR amplification with P23/P24 primer pair, lysC^{gla-T311I} was obtained by PCR amplification with P25/P26 primer pair, and the downstream homologous arm dn was obtained by PCR amplification with P27/P28 primer pair. The up-Psod segment was obtained by fusion PCR with P21/P24 primer pair using up and Psod as templates. The full-length segment up-Psod-lysC^{gla-T311I}-dn was obtained by fusion PCR with P21/P28 primer pair using up-Psod, lysC^{gla-T311I} and dn as templates. pK18mobsacB was digested with BamHI/HindIII. Enzyme-digested pK18mobsacB and up-Psod-lysC^{gla-T311I}-dn and were assembled using a seamless cloning kit and transformed into Trans1 T1 competent cells to obtain the recombinant plasmid pK18mobsacB-P_{sod}-*lysC*^{gla-T311I}-asd.

### 2. Construction of the plasmid pK18mobsacB-P_{sod}-aspB for enhancing expression of aspartate aminotransferase

The plasmid construction method was referred to above 1, and the primers used were P103, P104, P105, P106, P107 and P108.

### 3. Construction of plasmid pK18mobsacB-P_{cspB}-hom^{G378E}-thrB for enhancing expression of homoserine dehydrogenase-homoserine kinase operon

The plasmid construction method was referred to above 1, and the primers used were P29, P30, P31, P32, P33, P34, P35, and P36.

### 4. Construction of the plasmid pK18mobsacB-P_{sod}-thrC^{gla} for enhancing expression of threonine synthase

The plasmid construction method was referred to above 1, and the primers used were P37, P38, P39, P40, P41 and P42.

### 5. Construction of the plasmid pK18mobsacB-P_{sod}-rhtC for enhancing expression of threonine export protein

The plasmid construction method was referred to above 1, and the primers used were P157, P158, P159, P160, P161, P162, P163 and P164.

### 6. Construction of the plasmid pK18mobsacB-Ptuf-ppc^{D299N} for enhancing expression of phosphoenolpyruvate carboxylase

The upstream homologous arm up was obtained by PCR amplification with P53/P54 primer pair using ATCC13032 genome as template, the promoter segment Ptuf was obtained by PCR amplification with P55/P56 primer pair, ppc^{D299N} was obtained by PCR amplification with P57/P58 primer pair, and the downstream homologous arm dn was obtained by PCR amplification with P59/P60 primer pair. The segment up-Ptuf was obtained by fusion PCR with P53/P56 primer pair using up and Ptuf as templates. The full length segment up-Ptuf-ppc^{D299N}-dn was obtained by fusion PCR with P53/P60 primer pair using up-Ptuf, ppc^{D299N} and dn as templates. pK18mobsacB was digested with BamHI/HindIII. Enzyme-digested pK18mobsacB and up-Ptuf-ppc^{D299N}-dn were assembled using a seamless cloning kit and transformed into Trans1 T1 competent cells to obtain the recombinant plasmid pK18mobsacB-Ptuf-*ppc*^{D299N}.

The primers used in the above plasmid construction process are shown in Table 1.

**Table 1 Primer sequences**

| Name | Sequence (SEQ ID NOs: 7-50 in order) |
|---|---|
| P21 | AATTCGAGCTCGGTACCCGGGGATCCAGCGACAGGACAAGCACTGG |
| P22 | CCCGGAATAATTGGCAGCTATGTGCACCTTTCGATCTACG |
| P23 | CGTAGATCGAAAGGTGCACATAGCTGCCAATTATTCCGGG |
| P24 | TTTCTGTACGACCAGGGCCATGGGTAAAAAATCCTTTCGTA |
| P25 | TACGAAAGGATTTTTTACCCATGGCCCTGGTCGTACAGAAA |
| P26 | TCGGAACGAGGGCAGGTGAAGGTGATGTCGGTGGTGCCGTCT |
| P27 | AGACGGCACCACCGACATCACCTTCACCTGCCCTCGTTCCGA |
| P28 | GTAAAACGACGGCCAGTGCCAAGCTTAGCCTGGTAAGAGGAAACGT |
| P29 | AATTCGAGCTCGGTACCCGGGGATCCCTGCGGGCAGATCCTTTTGA |
| P30 | ATTTCTTTATAAACGCAGGTCATATCTACCAAAACTACGC |
| P31 | GCGTAGTTTTGGTAGATATGACCTGCGTTTATAAAGAAAT |
| P32 | GTATATCTCCTTCTGCAGGAATAGGTATCGAAAGACGAAA |
| P33 | TTTCGTCTTTCGATACCTATTCCTGCAGAAGGAGATATAC |
| P34 | TAGCCAATTCAGCCAAAACCCCCACGCGATCTTCCACATCC |
| P35 | GGATGTGGAAGATCGCGTGGGGGTTTTGGCTGAATTGGCTA |
| P36 | GTAAAACGACGGCCAGTGCCAAGCTTGCTGGCTCTTGCCGTCGATA |
| P37 | ATTCGAGCTCGGTACCCGGGGATCCGCCGTTGATCATTGTTCTTCA |
| P38 | CCCGGAATAATTGGCAGCTAGGATATAACCCTATCCCAAG |
| P39 | CTTGGGATAGGGTTATATCCTAGCTGCCAATTATTCCGGG |
| P40 | ACGCGTCGAAATGTAGTCCATGGGTAAAAAATCCTTTCGTA |
| P41 | TACGAAAGGATTTTTTACCCATGGACTACATTTCGACGCGT |
| P42 | GTAAAACGACGGCCAGTGCCAAGCTTGAATACGCGGATTCCCTCGC |
| P53 | AATTCGAGCTCGGTACCCGGGGATCCTACGTCGTCGAGCAGACCCG |
| P54 | CATTCGCAGGGTAACGGCCAAGGGTGTTGGCGTGCATGAG |
| P55 | CTCATGCACGCCAACACCCTTGGCCGTTACCCTGCGAATG |
| P56 | TCGCGTAAAAAATCAGTCATTGTATGTCCTCCTGGACTTC |
| P57 | GAAGTCCAGGAGGACATACAATGACTGATTTTTTACGCGA |
| P58 | GTGACCTTATTCATGCGGTTCGACAGGCTGAGCTCATGCT |
| P59 | AGCATGAGCTCAGCCTGTCGAACCGCATGAATAAGGTCAC |
| P60 | GTAAAACGACGGCCAGTGCCAAGCTTGGTGACTTGGGCGCGTTCGA |
| P103 | GAGCTCGGTACCCGGGGATCCGCAGGGTATTGCAGGGACTCA |
| P104 | CAAGCCCGGAATAATTGGCAGCTAAACTGCGTACCTCCGCATGTGGTGG |
| P105 | TAGCTGCCAATTATTCCGGGCTTGT |
| P106 | GGGTAAAAAATCCTTTCGTAGGTTT |
| P107 | GGAAACCTACGAAAGGATTTTTTACCCATGAGTTCAGTTTCGCTGCAGGATTT |
| P108 | ACGACGGCCAGTGCCAAGCTTACACCGGAACAACCCACATG |
| P157 | TACGAATTCGAGCTCGGTACCCGGGGATCCAGTTAACTCCACCGACCGGGTACTGC |
| P158 | AAGCCCGGAATAATTGGCAGCTATGTCTTCGCTGGACCAAGAG |
| P159 | CTCTTGGTCCAGCGAAGACATAGCTGCCAATTATTCCGGGCTT |
| P160 | GACGGTGAGAAATAACATCAACATGGGTAAAAAATCCTTTCGTA |
| P161 | TACGAAAGGATTTTTTACCCATGTTGATGTTATTTCTCACCGTC |
| P162 | TGCCTCTTTTAGCCTTTTCAGAGGGTCACCGCGAAATAATCAAATGAA |
| P163 | TTCATTTGATTATTTCGCGGTGACCCTCTGAAAAGGCTAAAAGAGGCA |
| P164 | GTTGTAAAACGACGGCCAGTGCCAAGCTTAAAAGGCAGTCCAGTACACCCT |

### Example 2 Construction of a genome-modified strain

### 1. Construction of a strain with enhanced expression of aspartate aminotransferase

ATCC13032 competent cells were prepared according to the classic method of Corynebacterium glutamicum (C. glutamicum Handbook, Charpter 23). The competent cells were transformed with the recombinant plasmid pK18mobsacB-P_{sod}-*aspB* by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The fragment of interest was amplified by PCR and analyzed by nucleotide sequencing to obtain the mutant strain of interest named SMCT061. Compared to strain ATCC13032, the promoter of the aspB gene in this strain was replaced with the Psod promoter.

### 2. Construction of a strain with enhanced expression of aspartate kinase-aspartate semialdehyde dehydrogenase operon

The strain construction method was referred to the above 1. SMCT061 was used as the original strain, and the pK18mobsacB-P_{sod}-*lysC*^{gla-T311I}-asd plasmid was introduced into SMCT061 to perform modification for enhancing aspartate kinase-aspartate semialdehyde dehydrogenase operon. The obtained strain was named SMCT062. Compared to strain SMCT061, the lysC gene of this strain was mutated, resulting in its start codon to mutate from GTG to ATG, and the position 311 of the encoded amino acid sequence to mutate from threonine to isoleucine, and the promoter of the lysC-asd operon was replaced with the Psod promoter.

### 3. Construction of a strain with enhanced expression of homoserine dehydrogenase-homoserine kinase

The strain construction method was referred to the above 1. SMCT062 was used as the original strain, and the pK18mobsacB-P_{cspB}-*hom*^{G378E}-*thrB* plasmid was introduced into SMCT062 to perform modification for enhancing the expression of homoserine dehydrogenase-homoserine kinase. The obtained modified strain was named SMCT063. Compared to the original strain SMCT062, the horn gene of this strain was mutated, resulting in the G378E mutation in its encoded protein, and the promoter of the hom-thrB operon was replaced with P_{cspB} promoter.

### 4. Construction of a strain with enhanced expression of threonine synthase

The strain construction method was referred to the above 1. SMCT063 was used as the original strain, and the pK18mobsacB-P_{sod}-*thrC*^{gla} plasmid was introduced into SMCT063 to perform modification for enhancing the expression of threonine synthase. The obtained modified strain was named SMCT064. Compared to the strain SMCT063, the start codon of the thrC gene of this strain was mutated to ATG, and the promoter of the thrC gene was replaced with P_{sod}.

### 5. Construction of a strain with enhanced expression of threonine export protein

The strain construction method was referred to the above 1. SMCT064 was used as the original strain, and the pK18mobsacB-P_{sod}-*rhtC* plasmid was introduced into SMCT064 to perform modification for enhancing the expression of threonine export protein. The obtained modified strain was named SMCT065. Compared to SMCT064, the threonine export protein gene *rhtC* from Escherichia coli was inserted downstream of the cg2009 gene in this strain.

### 6. Construction of a strain with enhanced expression of phosphoenolpyruvate carboxylase

The strain construction method was referred to the above 1. SMCT061, SMCT062, SMCT063, SMCT064, and SMCT065 were used as original strains, and the pK18mobsacB-Ptuf-*ppc*^{D299N} plasmid was introduced into the above original strains, respectively, to perform modification for enhancing the expression of phosphoenolpyruvate carboxylase. The obtained modified strains were named SMCT066, SMCT067, SMCT068, SMCT079, and SMCT070, respectively. Compared to their corresponding original strains, the mutation of the gene ppc encoding phosphoenolpyruvate carboxylase in these modified strains caused the D299N mutation in the proteins encoded, and the 27 bp segment upstream of the start codon of the ppc gene was replaced with Ptuf promoter.

The genotypes of the above-mentioned strains obtained by genetic modification are shown in Table 2.

**Table 2 Genotype information of strains**

| Strains | Genotype |
|---|---|
| SMCT061 | ATCC13032,Psod-*aspB* |
| SMCT062 | ATCC13032,Psod-*aspB*,P_{sod}-*lysC*^{gla-T311I}-*asd* |
| SMCT063 | ATCC13032,Psod-*aspB*,P_{sod}-*lysC*^{gla-T311I}-*asd,PcspB*-*hom^{G378E}*-*thrB* |
| SMCT064 | ATCC13032,Psod-*aspB*,P_{sod}-*lysC*^{gla-T311I}-*asd*,PcspB-*hom^{G378E}-thrB*,Psod-*thrC^{gla}* |
| SMCT065 | ATCC13032,Psod-*aspB*,P_{sod}-*lysC*^{,gla-T311I}-*asd*,PcspB-*hom^{G378E}-thrB*,Psod-*thrC^{gla}*,Psod-*rhtC* |
| SMCT066 | ATCC13032,Psod-*aspB*,Ptuf-*ppc*^{D299N} |
| SMCT067 | ATCC13032,Psod-*aspB*,P_{sod}-*lysC*^{gla-T311I}-*asd*,Ptuf-*ppc*^{D299N} |
| SMCT068 | ATCC13032,Psod-*aspB*,P_{sod}-*lysC*^{gla-T311I}-*asd*,*PcspB*-*hom^{G378E}*-*thrB*,Ptuf-*ppc*^{D299N} |
| SMCT069 | ATCC13032,Psod-*aspB*,P_{sod}-*lysC*^{gla-T311I}-*asd*,PcspB-*hom^{G378E}-thrB*,Psod-*thrC^{gla},*Ptuf-*ppc*^{D299N} |
| SMCT070 | ATCC13032,Psod-*aspB*,P_{sod}-*lysC*^{gla-T311I}-*asd*,PcspB-*hom^{G378E}*-*thrB*,Psod-*thrC^{gla}*,Psod-*rhtC*,Ptuf-*ppc*^{D299N} |

### Example 3 Shake flask fermentation verification of strains

Each of the modified strains constructed in Example 2 was validated by shake flask fermentation as follows:

### 1. Medium

Seed activation medium: BHI 3.7%, agar 2%, pH 7.

Seed medium: Peptone 5/L, yeast extract 5 g/L, sodium chloride 10 g/L, ammonium sulfate 16 g/L, urea 8 g/L, potassium dihydrogen phosphate 10.4 g/L, dipotassium hydrogen phosphate 21.4 g/L, biotin 5 mg/L, magnesium sulfate 3 g/L. Glucose 50 g/L, pH 7.2.

Fermentation medium: corn steep liquor 50 mL/L, glucose 30 g/L, ammonium sulfate 4 g/L, MOPS 30 g/L, potassium dihydrogen phosphate 10 g/L, urea 20 g/L, biotin 10 mg/L, magnesium sulfate 6 g/L, ferrous sulfate 1 g/L, VB1•HCl 40 mg/L, calcium pantothenate 50 mg/L, nicotinamide 40 mg/L, manganese sulfate 1 g/L, zinc sulfate 20 mg/L, copper sulfate 20 mg/L, pH 7.2.

### 2. Production of L-threonine by shake flask fermentation with engineered strain

(1) Seed culture: 1 loop of seed of SMCT061, SMCT062, SMCT063, SMCT064, SMCT065, SMCT066, SMCT067, SMCT068, SMCT069 and SMCT070 on the slant culture medium was picked and inoculated into a 500 mL Erlenmeyer flask containing 20 mL of seed culture medium, and cultured at 30°C and 220 r/min for 16 h to obtain a seed broth.
(2) Fermentation culture: 2 mL of seed liquid was inoculated into a 500 mL Erlenmeyer flask containing 20 mL of fermentation medium and cultured at 33°C and 220 r/min under shaking for 24 h to obtain a fermentation broth.
(3) 1 mL of fermentation broth was taken and centrifuged (12000 rpm, 2 min), and the supernatant was collected. L-threonine in the fermentation broth of modified strain and control strain were detected by HPLC.

The results of shake flask fermentation for threonine are shown in Table 3.

**Table 3 Fermentation test results**

| Chassis strain | | | ppc modified strain | | |
|---|---|---|---|---|---|
| Strain number | OD₅₆₂ | Threonine production (g/L) | Strain number | OD₅₆₂ | Threonine production (g/L) |
| SMCT061 | 24 | 0.8 | SMCT066 | 24 | 1.0 |
| SMCT062 | 23 | 2.5 | SMCT067 | 23 | 3.3 |
| SMCT063 | 23 | 3.8 | SMCT068 | 23 | 5.1 |
| SMCT064 | 22 | 5.0 | SMCT069 | 22 | 6.9 |
| SMCT065 | 22 | 6.2 | SMCT070 | 22 | 8.7 |

The results showed that by optimizing the promoter of the gene encoding phosphoenolpyruvate carboxylase and engineering the D299N mutation on phosphoenolpyruvate carboxylase in the threonine-producing strains SMCT061, SMCT062, SMCT063, SMCT064, and SMCT065, the threonine production was further increased. The threonine production of strains SMCT066, SMCT067, SMCT068, SMCT069, and SMCT070 was increased by 25%, 33%, 35%, 38%, and 40%, respectively, indicating that by replacing the 20-30 bp segment upstream of the start codon of the gene encoding phosphoenolpyruvate carboxylase with a strong promoter and engineering the D299N mutation on phosphoenolpyruvate carboxylase, the supply of oxaloacetate, a precursor of threonine synthesis, could be increased, thereby promoting the synthesis of threonine. In addition, the threonine production was further increased by combining the modification of the above-mentioned phosphoenolpyruvate carboxylase with the enhanced expression of different enzymes in the threonine synthesis pathway and threonine export proteins, indicating that the combination of the above-mentioned modifications was more conducive to improving the threonine-producing ability of the strain.

Although the present invention has been described in detail above with general descriptions and specific embodiments, it is obvious to those skilled in the art that some modifications or improvements may be made based on the present invention. Therefore, these modifications or improvements made without departing from the spirit of the present invention belong to the scope of protection claimed by the present invention.

### Description of sequences

SEQ ID No:1 Corynebacterium glutamicum PPC wt
Met Thr Asp Phe Leu Arg Asp Asp Ile Arg Phe Leu Gly Gln Ile Leu
1               5                   10                  15
Gly Glu Val Ile Ala Glu Gln Glu Gly Gln Glu Val Tyr Glu Leu Val

20                  25                  30
Glu Gln Ala Arg Leu Thr Ser Phe Asp Ile Ala Lys Gly Asn Ala Glu
        35                  40                  45
Met Asp Ser Leu Val Gln Val Phe Asp Gly Ile Thr Pro Ala Lys Ala
    50                  55                  60
Thr Pro Ile Ala Arg Ala Phe Ser His Phe Ala Leu Leu Ala Asn Leu
65                  70                  75                  80
Ala Glu Asp Leu Tyr Asp Glu Glu Leu Arg Glu Gln Ala Leu Asp Ala
                85                  90                  95
Gly Asp Thr Pro Pro Asp Ser Thr Leu Asp Ala Thr Trp Leu Lys Leu
            100                 105                 110
Asn Glu Gly Asn Val Gly Ala Glu Ala Val Ala Asp Val Leu Arg Asn
        115                 120                 125
Ala Glu Val Ala Pro Val Leu Thr Ala His Pro Thr Glu Thr Arg Arg
    130                 135                 140
Arg Thr Val Phe Asp Ala Gln Lys Trp Ile Thr Thr His Met Arg Glu
145                 150                 155                 160
Arg His Ala Leu Gln Ser Ala Glu Pro Thr Ala Arg Thr Gln Ser Lys
                165                 170                 175
Leu Asp Glu Ile Glu Lys Asn Ile Arg Arg Arg Ile Thr Ile Leu Trp
            180                 185                 190
Gln Thr Ala Leu Ile Arg Val Ala Arg Pro Arg Ile Glu Asp Glu Ile
        195                 200                 205
Glu Val Gly Leu Arg Tyr Tyr Lys Leu Ser Leu Leu Glu Glu Ile Pro
    210                 215                 220
Arg Ile Asn Arg Asp Val Ala Val Glu Leu Arg Glu Arg Phe Gly Glu
225                 230                 235                 240
Gly Val Pro Leu Lys Pro Val Val Lys Pro Gly Ser Trp Ile Gly Gly
                245                 250                 255
Asp His Asp Gly Asn Pro Tyr Val Thr Ala Glu Thr Val Glu Tyr Ser
            260                 265                 270
Thr His Arg Ala Ala Glu Thr Val Leu Lys Tyr Tyr Ala Arg Gln Leu
        275                 280                 285
His Ser Leu Glu His Glu Leu Ser Leu Ser Asp Arg Met Asn Lys Val
    290                 295                 300
Thr Pro Gln Leu Leu Ala Leu Ala Asp Ala Gly His Asn Asp Val Pro
305                 310                 315                 320
Ser Arg Val Asp Glu Pro Tyr Arg Arg Ala Val His Gly Val Arg Gly
                325                 330                 335
Arg Ile Leu Ala Thr Thr Ala Glu Leu Ile Gly Glu Asp Ala Val Glu
            340                 345                 350
Gly Val Trp Phe Lys Val Phe Thr Pro Tyr Ala Ser Pro Glu Glu Phe
        355                 360                 365
Leu Asn Asp Ala Leu Thr Ile Asp His Ser Leu Arg Glu Ser Lys Asp
    370                 375                 380
Val Leu Ile Ala Asp Asp Arg Leu Ser Val Leu Ile Ser Ala Ile Glu
385                 390                 395                 400

Ser Phe Gly Phe Asn Leu Tyr Ala Leu Asp Leu Arg Gln Asn Ser Glu
                405                 410                 415
Ser Tyr Glu Asp Val Leu Thr Glu Leu Phe Glu Arg Ala Gln Val Thr
            420                 425                 430
Ala Asn Tyr Arg Glu Leu Ser Glu Ala Glu Lys Leu Glu Val Leu Leu
        435                 440                 445
Lys Glu Leu Arg Ser Pro Arg Pro Leu Ile Pro His Gly Ser Asp Glu
    450                 455                 460
Tyr Ser Glu Val Thr Asp Arg Glu Leu Gly Ile Phe Arg Thr Ala Ser
465                 470                 475                 480
Glu Ala Val Lys Lys Phe Gly Pro Arg Met Val Pro His Cys Ile Ile
                485                 490                 495
Ser Met Ala Ser Ser Val Thr Asp Val Leu Glu Pro Met Val Leu Leu
            500                 505                 510
Lys Glu Phe Gly Leu Ile Ala Ala Asn Gly Asp Asn Pro Arg Gly Thr
        515                 520                 525
Val Asp Val Ile Pro Leu Phe Glu Thr Ile Glu Asp Leu Gln Ala Gly
    530                 535                 540
Ala Gly Ile Leu Asp Glu Leu Trp Lys Ile Asp Leu Tyr Arg Asn Tyr
545                 550                 555                 560
Leu Leu Gln Arg Asp Asn Val Gln Glu Val Met Leu Gly Tyr Ser Asp
                565                 570                 575
Ser Asn Lys Asp Gly Gly Tyr Phe Ser Ala Asn Trp Ala Leu Tyr Asp
            580                 585                 590
Ala Glu Leu Gln Leu Val Glu Leu Cys Arg Ser Ala Gly Val Lys Leu
        595                 600                 605
Arg Leu Phe His Gly Arg Gly Gly Thr Val Gly Arg Gly Gly Gly Pro
    610                 615                 620
Ser Tyr Asp Ala Ile Leu Ala Gln Pro Arg Gly Ala Val Gln Gly Ser
625                 630                 635                 640
Val Arg Ile Thr Glu Gln Gly Glu Ile Ile Ser Ala Lys Tyr Gly Asn
                645                 650                 655
Pro Glu Thr Ala Arg Arg Asn Leu Glu Ala Leu Val Ser Ala Thr Leu
            660                 665                 670
Glu Ala Ser Leu Leu Asp Val Ser Glu Leu Thr Asp His Gln Arg Ala
        675                 680                 685
Tyr Asp Ile Met Ser Glu Ile Ser Glu Leu Ser Leu Lys Lys Tyr Ala
    690                 695                 700
Ser Leu Val His Glu Asp Gln Gly Phe Ile Asp Tyr Phe Thr Gln Ser
705                 710                 715                 720
Thr Pro Leu Gln Glu Ile Gly Ser Leu Asn Ile Gly Ser Arg Pro Ser
                725                 730                 735
Ser Arg Lys Gln Thr Ser Ser Val Glu Asp Leu Arg Ala Ile Pro Trp
            740                 745                 750
Val Leu Ser Trp Ser Gln Ser Arg Val Met Leu Pro Gly Trp Phe Gly
        755                 760                 765
Val Gly Thr Ala Leu Glu Gln Trp Ile Gly Glu Gly Glu Gln Ala Thr

770                 775                 780
Gln Arg Ile Ala Glu Leu Gln Thr Leu Asn Glu Ser Trp Pro Phe Phe
785                 790                 795                 800
Thr Ser Val Leu Asp Asn Met Ala Gln Val Met Ser Lys Ala Glu Leu
                805                 810                 815
Arg Leu Ala Lys Leu Tyr Ala Asp Leu Ile Pro Asp Thr Glu Val Ala
            820                 825                 830
Glu Arg Val Tyr Ser Val Ile Arg Glu Glu Tyr Phe Leu Thr Lys Lys
        835                 840                 845
Met Phe Cys Val Ile Thr Gly Ser Asp Asp Leu Leu Asp Asp Asn Pro
    850                 855                 860
Leu Leu Ala Arg Ser Val Gln Arg Arg Tyr Pro Tyr Leu Leu Pro Leu
865                 870                 875                 880
Asn Val Ile Gln Val Glu Met Met Arg Arg Tyr Arg Lys Gly Asp Gln
                885                 890                 895
Ser Glu Gln Val Ser Arg Asn Ile Gln Leu Thr Met Asn Gly Leu Ser
            900                 905                 910
Thr Ala Leu Arg Asn Ser Gly
        915
SEQ ID No:2 Corynebacterium glutamicum PPC D299N
Met Thr Asp Phe Leu Arg Asp Asp Ile Arg Phe Leu Gly Gln Ile Leu
1               5                   10                  15
Gly Glu Val Ile Ala Glu Gln Glu Gly Gln Glu Val Tyr Glu Leu Val
            20                  25                  30
Glu Gln Ala Arg Leu Thr Ser Phe Asp Ile Ala Lys Gly Asn Ala Glu
        35                  40                  45
Met Asp Ser Leu Val Gln Val Phe Asp Gly Ile Thr Pro Ala Lys Ala
    50                  55                  60
Thr Pro Ile Ala Arg Ala Phe Ser His Phe Ala Leu Leu Ala Asn Leu
65                  70                  75                  80
Ala Glu Asp Leu Tyr Asp Glu Glu Leu Arg Glu Gln Ala Leu Asp Ala
                85                  90                  95
Gly Asp Thr Pro Pro Asp Ser Thr Leu Asp Ala Thr Trp Leu Lys Leu
            100                 105                 110
Asn Glu Gly Asn Val Gly Ala Glu Ala Val Ala Asp Val Leu Arg Asn
        115                 120                 125
Ala Glu Val Ala Pro Val Leu Thr Ala His Pro Thr Glu Thr Arg Arg
    130                 135                 140
Arg Thr Val Phe Asp Ala Gln Lys Trp Ile Thr Thr His Met Arg Glu
145                 150                 155                 160
Arg His Ala Leu Gln Ser Ala Glu Pro Thr Ala Arg Thr Gln Ser Lys
                165                 170                 175
Leu Asp Glu Ile Glu Lys Asn Ile Arg Arg Arg Ile Thr Ile Leu Trp
            180                 185                 190
Gln Thr Ala Leu Ile Arg Val Ala Arg Pro Arg Ile Glu Asp Glu Ile
        195                 200                 205
Glu Val Gly Leu Arg Tyr Tyr Lys Leu Ser Leu Leu Glu Glu Ile Pro

210                 215                 220
Arg Ile Asn Arg Asp Val Ala Val Glu Leu Arg Glu Arg Phe Gly Glu
225                 230                 235                 240
Gly Val Pro Leu Lys Pro Val Val Lys Pro Gly Ser Trp Ile Gly Gly
                245                 250                 255
Asp His Asp Gly Asn Pro Tyr Val Thr Ala Glu Thr Val Glu Tyr Ser
            260                 265                 270
Thr His Arg Ala Ala Glu Thr Val Leu Lys Tyr Tyr Ala Arg Gln Leu
        275                 280                 285
His Ser Leu Glu His Glu Leu Ser Leu Ser Asn Arg Met Asn Lys Val
    290                 295                 300
Thr Pro Gln Leu Leu Ala Leu Ala Asp Ala Gly His Asn Asp Val Pro
305                 310                 315                 320
Ser Arg Val Asp Glu Pro Tyr Arg Arg Ala Val His Gly Val Arg Gly
                325                 330                 335
Arg Ile Leu Ala Thr Thr Ala Glu Leu Ile Gly Glu Asp Ala Val Glu
            340                 345                 350
Gly Val Trp Phe Lys Val Phe Thr Pro Tyr Ala Ser Pro Glu Glu Phe
        355                 360                 365
Leu Asn Asp Ala Leu Thr Ile Asp His Ser Leu Arg Glu Ser Lys Asp
    370                 375                 380
Val Leu Ile Ala Asp Asp Arg Leu Ser Val Leu Ile Ser Ala Ile Glu
385                 390                 395                 400
Ser Phe Gly Phe Asn Leu Tyr Ala Leu Asp Leu Arg Gln Asn Ser Glu
                405                 410                 415
Ser Tyr Glu Asp Val Leu Thr Glu Leu Phe Glu Arg Ala Gln Val Thr
            420                 425                 430
Ala Asn Tyr Arg Glu Leu Ser Glu Ala Glu Lys Leu Glu Val Leu Leu
        435                 440                 445
Lys Glu Leu Arg Ser Pro Arg Pro Leu Ile Pro His Gly Ser Asp Glu
    450                 455                 460
Tyr Ser Glu Val Thr Asp Arg Glu Leu Gly Ile Phe Arg Thr Ala Ser
465                 470                 475                 480
Glu Ala Val Lys Lys Phe Gly Pro Arg Met Val Pro His Cys Ile Ile
                485                 490                 495
Ser Met Ala Ser Ser Val Thr Asp Val Leu Glu Pro Met Val Leu Leu
            500                 505                 510
Lys Glu Phe Gly Leu Ile Ala Ala Asn Gly Asp Asn Pro Arg Gly Thr
        515                 520                 525
Val Asp Val Ile Pro Leu Phe Glu Thr Ile Glu Asp Leu Gln Ala Gly
    530                 535                 540
Ala Gly Ile Leu Asp Glu Leu Trp Lys Ile Asp Leu Tyr Arg Asn Tyr
545                 550                 555                 560
Leu Leu Gln Arg Asp Asn Val Gln Glu Val Met Leu Gly Tyr Ser Asp
                565                 570                 575
Ser Asn Lys Asp Gly Gly Tyr Phe Ser Ala Asn Trp Ala Leu Tyr Asp
            580                 585                 590

Ala Glu Leu Gln Leu Val Glu Leu Cys Arg Ser Ala Gly Val Lys Leu
        595                 600                 605
Arg Leu Phe His Gly Arg Gly Gly Thr Val Gly Arg Gly Gly Gly Pro
    610                 615                 620
Ser Tyr Asp Ala Ile Leu Ala Gln Pro Arg Gly Ala Val Gln Gly Ser
625                 630                 635                 640
Val Arg Ile Thr Glu Gln Gly Glu Ile Ile Ser Ala Lys Tyr Gly Asn
                645                 650                 655
Pro Glu Thr Ala Arg Arg Asn Leu Glu Ala Leu Val Ser Ala Thr Leu
            660                 665                 670
Glu Ala Ser Leu Leu Asp Val Ser Glu Leu Thr Asp His Gln Arg Ala
        675                 680                 685
Tyr Asp Ile Met Ser Glu Ile Ser Glu Leu Ser Leu Lys Lys Tyr Ala
    690                 695                 700
Ser Leu Val His Glu Asp Gln Gly Phe Ile Asp Tyr Phe Thr Gln Ser
705                 710                 715                 720
Thr Pro Leu Gln Glu Ile Gly Ser Leu Asn Ile Gly Ser Arg Pro Ser
                725                 730                 735
Ser Arg Lys Gln Thr Ser Ser Val Glu Asp Leu Arg Ala Ile Pro Trp
            740                 745                 750
Val Leu Ser Trp Ser Gln Ser Arg Val Met Leu Pro Gly Trp Phe Gly
        755                 760                 765
Val Gly Thr Ala Leu Glu Gln Trp Ile Gly Glu Gly Glu Gln Ala Thr
    770                 775                 780
Gln Arg Ile Ala Glu Leu Gln Thr Leu Asn Glu Ser Trp Pro Phe Phe
785                 790                 795                 800
Thr Ser Val Leu Asp Asn Met Ala Gln Val Met Ser Lys Ala Glu Leu
                805                 810                 815
Arg Leu Ala Lys Leu Tyr Ala Asp Leu Ile Pro Asp Thr Glu Val Ala
            820                 825                 830
Glu Arg Val Tyr Ser Val Ile Arg Glu Glu Tyr Phe Leu Thr Lys Lys
        835                 840                 845
Met Phe Cys Val Ile Thr Gly Ser Asp Asp Leu Leu Asp Asp Asn Pro
    850                 855                 860
Leu Leu Ala Arg Ser Val Gln Arg Arg Tyr Pro Tyr Leu Leu Pro Leu
865                 870                 875                 880
Asn Val Ile Gln Val Glu Met Met Arg Arg Tyr Arg Lys Gly Asp Gln
                885                 890                 895
Ser Glu Gln Val Ser Arg Asn Ile Gln Leu Thr Met Asn Gly Leu Ser
            900                 905                 910
Thr Ala Leu Arg Asn Ser Gly
        915
SEQ ID No:3 27bp Up
caatgtgaaa gagtgtttaa agtagtt                                        27
SEQ ID No: 4 Ptuf
tggccgttac cctgcgaatg tccacagggt agctggtagt ttgaaaatca acgccgttgc      60
ccttaggatt cagtaactgg cacattttgt aatgcgctag atctgtgtgc tcagtcttcc     120

aggctgctta tcacagtgaa agcaaaacca attcgtggct gcgaaagtcg tagccaccac 180
gaagtccagg aggacataca 200
SEQ ID No: 5 Psod
tagctgccaa ttattccggg cttgtgaccc gctacccgat aaataggtcg gctgaaaaat 60
ttcgttgcaa tatcaacaaa aaggcctatc attgggaggt gtcgcaccaa gtacttttgc 120
gaagcgccat ctgacggatt ttcaaaagat gtatatgctc ggtgcggaaa cctacgaaag 180
gattttttac cc 192
SEQ ID No: 6 PcspB
acctgcgttt ataaagaaat gtaaacgtga tcggatcgat ataaaagaaa cagtttgtac 60
tcaggtttga agcattttct ccaattcgcc tggcaaaaat ctcaattgtc gcttacagtt 120
tttctcaacg acaggctgct aagctgctag ttcggtggcc tagtgagtgg cgtttacttg 180
gataaaagta atcccatgtc gtgatcagcc attttgggtt gtttccatag catccaaagg 240
tttcgtcttt cgatacctat 260

## Claims

1. Use of enhanced expression of a gene encoding phosphoenolpyruvate carboxylase in increasing the production of threonine production by a *Corynebacterium* species or in constructing a *Corynebacterium* species that produces threonine,
wherein the enhanced expression is achieved by replacing the 20-30 bp segment upstream of the start codon of the gene encoding phosphoenolpyruvate carboxylase with a strong promoter.

2. The use of claim 1, wherein the enhanced expression is achieved by replacing the 27 bp segment upstream of the start codon of the gene encoding phosphoenolpyruvate carboxylase with a strong promoter;
preferably, the strong promoter is Ptuf.

3. The use of claim 1 or 2, wherein the amino acid sequence of the phosphoenolpyruvate carboxylase is SEQ ID NO. 1 or 2.

4. A recombinant microorganism, wherein, the 20-30 bp segment upstream of the start codon of a gene encoding phosphoenolpyruvate carboxylase in the recombinant microorganism is replaced with a strong promoter;
preferably, wherein the 27 bp segment upstream of the start codon of a gene encoding phosphoenolpyruvate carboxylase in the recombinant microorganism is replaced with a strong promoter;
more preferably, the strong promoter is Ptuf.

5. The recombinant microorganism of claim 4, wherein the amino acid sequence of the phosphoenolpyruvate carboxylase of the recombinant microorganism is SEQ ID NO. 1 or 2.

6. The recombinant microorganism of claim 4 or 5, wherein the enzyme activity of any one or more of the following enzymes (1) to (7) in the recombinant microorganism is enhanced and/or the feedback inhibition thereof is deregulated:
(1) aspartate kinase;
(2) aspartate semialdehyde dehydrogenase;
(3) homoserine dehydrogenase;
(4) threonine synthase;
(5) homoserine kinase;
(6) aspartate aminotransferase; and
(7) threonine export protein;
preferably, the threonine export protein is one derived from *Escherichia coli.*

7. The recombinant microorganism of claim 6, wherein the enhancement of the enzyme activity is achieved by any one of or any combination of 1) to 6) below:
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme.

8. The recombinant microorganism of any one of claims 4 to 7, wherein the starting microorganism used for constructing the recombinant microorganism is a *Corynebacterium* species, preferably *Corynebacterium glutamicum.*

9. Use of the recombinant microorganism of any one of claims 4 to 8 in any one of:
(1) fermentative production of threonine or a derivative thereof;
(2) as a starting strain, engineering the strain producing threonine or a derivative thereof; and
(3) increasing the production and/or yield of threonine or a derivative thereof.

10. A method for fermentative production of threonine or a derivative thereof, comprising a step of culturing the recombinant microorganism of any one of claims 4 to 8, and isolating threonine or the derivative thereof from the culture.
